# EUROPÄISCHE PATENTSCHRIFT

(11) **EP 1 919 436 B1**
(45) Veröffentlichungstag und Bekanntmachung des Hinweises auf die Patenterteilung: **17.11.2010**
(21) Anmeldenummer: 06775775.7
(22) Anmeldetag: 27.07.2006
(51) Int. Cl.: A61K 6/097, A61K 6/083, A61L 27/34

(54) **WERKSTOFF FÜR DEN ÜBERWIEGEND MEDIZINISCHEN, LANGFRISTIGEN IN-VIVO EINSATZ**
MATERIAL PRIMARILY FOR MEDICAL, LONG-TERM IN VIVO USE, AND METHOD FOR THE PRODUCTION THEREOF
MATERIAU PREVU POUR L'UTILISATION PRINCIPALEMENT MEDICALE, IN VIVO A LONG TERME, ET PROCEDE DE REALISATION

(30) Priorität: 01.09.2005 DE 102005042078
(43) Veröffentlichungstag der Anmeldung: 14.05.2008
(73) Patentinhaber: BioCer Entwicklungs-GmbH, 95447 Bayreuth (DE); Klinikum der Universität Regensburg, 93042 Regensburg (DE)
(72) Erfinder: KOKOTT, Andreas, 95138 Bad Steben (DE); HOFFMANN, Bettina, 95445 Bayreuth (DE); ZIEGLER, Günter, 95488 Eckersdorf (DE); BEHR, Michael, 93053 Regensburg (DE); ROSENTRITT, Martin, 93053 Regensburg (DE)
(74) Vertreter: Donath, Dirk
(86) Internationale Anmeldenummer: PCT/DE2006/001334
(87) Internationale Veröffentlichungsnummer: WO 2007/025496

(56) Entgegenhaltungen:
- GB-A- 1 433 303
- US-A1- 2005 118 239
- DATABASE WPI Week 199814 Derwent Publications Ltd., London, GB; AN 1998-153970 XP002452887 & JP 10 025218 A (KURARAY CO LTD) 27. Januar 1998 (1998-01-27)

## Beschreibung

Die Erfindung betrifft einen Werkstoff für den überwiegend medizinischen, langfristigen in-vivo Einsatz, wie bspw. als Füllungswerkstoff in der Zahnmedizin sowie ein Verfahren zu seiner Herstellung.

Es ist bekannt, dass körperfremde Materialien, die im Körper (z.B. in der Mundhöhle) oder am Körper (z.B. als Katheder) zur Anwendung kommen, den dort vorhandenen Mikroorganismen ausgesetzt sind oder das Eindringen von Mikroorganismen in den Körper begünstigen können.

Dabei handelt es sich bspw. in der Mundhöhle um eine aerobe oder anaerobe Mischflora. Zu dem am häufigsten dort auftretenden Bakterienstämmen gehören der Kariesauslösende *Streptococcus mutans* [Hellwig E et al. Einführung in die Zahnerhaltung. Urban & Schwarzenberg Verlag, München 1995] und als ein Erstbesiedler der *Streptococcus sanguis.*

Zu den häufig in der Mundhöhle eingesetzten Materialien zählen Metalle, Keramiken, Polymere oder auch Mischwerkstoffe, sog. Komposite [Eichner K. Zahnärztliche Werkstoffe und ihre Verarbeitung. Band 1, Band 2. Hüthig Verlag Heidelberg 1988 und Craig GC, Powers JM. Restorative Dental Materials. 11th ed. Mosby, St. Louis 2002].

Von allen bekannten dentalen Werkstoffen stehen die Komposite, die als Befestigungswerkstoffe oder Füllungsmaterialien eingesetzt werden im Ruf, Bakterienanlagerungen in der Mundhöhle besonders zu begünstigen [Weitmann RT, Eames WB. Plaque accumulation on composite surfaces after various finishing procedures. J Am Dent Assoc 1975;91:101-106; Skorland KR, Sonju T. Effect of sucrose rinses on bacterial colonization on amalgam and composite. Acta Odontol Scand 1982;40:193-196 und Svanberg M, et al. Mutans streptococci in plaque from margins of amalgam, composite, and glass-ionmer restorations. J Dent Res 1990;69:861-864]. Erschwerend kommt hinzu, dass Komposite beim Polymerisieren schrumpfen, wodurch bei Füllungen oder Zementfugen mikrofeine Spalten zwischen Zahnsubstanz (Dentin/Schmelz) und dem Komposit entstehen können. Diesen feinen Spalt können Bakterien erfolgreich besiedeln [Hellwig E et al. Einführung in die Zahnerhaltung. Urban & Schwarzenberg Verlag, München 1995].

Da diese Spalträume der Zahnreinigung und der Spülwirkung des Speichels weitgehend entzogen sind, vermehren sich die Bakterien ungestört und führen nach kurzer Zeit zur Entstehung kariöser Läsionen. Bakterien können nicht nur Werkstoffe besiedeln, sie können auch die Kohlenstoffanteile der Polymere teilweise für ihren Stoffwechsel nutzen und so zum Abbau der Komposite beitragen [und Craig GC, Powers JM. Restorative Dental Materials. 11th ed. Mosby, St. Louis 2002].

Bakterien schaden also auf zweierlei Weise: Ihre ungestörte Vermehrung führt einerseits zu Karies und andererseits tragen sie zur allmählichen Zerstörung des Werkstoffs bei.

Die Freisetzung von Wirkstoffen aus medizinisch anwendbaren Werkstoffen ist schon seit mehreren Jahrzehnten bekannt. Applikationsorte sind u.a. Blutgefässe (Wirkstoff-Releasing aus beschichteten Stents zur Gefäßerweiterung) oder Knochen (bei Knocheninfektionen Implantation einer Polymer-Kugelkette aus Polymethylmethacrylat (Septopal® der Firma biometmerck) mit dem Antibiotikum Gentamycin (Refobacin® der Firma Merck).

Werden künstliche Hüftgelenke über eine "Zementierung" implantiert, wird auch bei dieser Vorgehensweise der "Zement" (aushärtende Polymermasse) mit einem Antibiotikum versetzt.

Während das Releasing aus den Stents die Restenose, also das Zuwachsen des Gefäßes verhindern soll, werden die Kugelketten bei bestehenden Infektionen eingesetzt. Im Falle der Hüftimplantation wird das Antibiotikum prophylaktisch eingesetzt, um die Entstehung einer Infektion zu verhüten.

In der Mundhöhle werden Systeme mit Wirkstoffen in Form von Mundspüllösungen, Zahnpasten verwendet [Lahdenperä MS, Puska MA, Alander PM, Waltimo T, Vallittu PK. Release of chlorhexidine diglugonate and flexural properties of glass fibre reinforced provisional fixed partial denture polymer. J Mat Sci Mat Med 2004;15:1349-1353; Imazato S. Influence of incorporation of antibacterial monomer on curing behaviour of a dental composite. J Dent 1999 ,27:292-297; Imazato S, Torii M. Incorporation of bacterial inhibitor into resin composite. J Dent Res 1994;73:1437-1444 und Addy M, Handley R. The effect of the incorporation of chlorhexidine acetate on some physical properties of polymerized and plasticized acrylics. J Oral Rehabil 1981;8.155-163].

Einer der häufigsten oralen antibakteriellen Wirkstoffe ist das Chlorhexidin-Digluconat [Lahdenperä MS, Puska MA, Alander PM, Waltimo T, Vallittu PK. Release of chlorhexidine diglugonate and flexural properties of glass fibre reinforced provisional fixed partial denture polymer. J Mat Sci Mat Med 2004;15:1349-1353]. Bei Anwendungen über mehr als sechs Wochen kommt es zu Schleimhautverfärbungen und Geschmacksirritationen, weswegen eine Dauermedikation nicht sinnvoll ist.

Von dentalen Amalgamen weiß man, dass die Freisetzung flüchtiger Bestandteile wie z.B. Kupfer im Füllungsspalt Mikroorganismen das Überleben erschwert oder unmöglich macht [Skorland KR, Sonju T. Effect of sucrose rinses on bacterial colonization on amalgam and composite. Acta Odontol Scand 1982;40:193-196 und Svanberg M, et al. Mutans streptococci in plaque from margins of amalgam, composite, and glass-ionmer restorations. J Dent Res 1990;69:861-864].

Für Komposite werden Konzepte diskutiert, bei denen durch Einlagerung freisetzbarer bakterizid wirkender Substanzen die Plaqueanlagerung verringert oder gar verhindert werden soll [Imazato S. Influence of incorporation of antibacterial monomer on curing behaviour of a dental composite. J Dent 1999 ,27:292-297 und Imazato S, Torii M. Incorporation of bacterial inhibitor into resin composite. J Dent Res 1994;73:1437-1444].

Der Nachteil aller voran stehenden Lösungen besteht jedoch darin, dass viele der prinzipiell in Frage kommenden Substanzen mit antibiotischer Wirkung allergische oder toxische Wirkungen entfalten können. Darüber hinaus muss bei den bekannten Werkstoffen (bspw. Zement oder Füllung) sichergestellt sein, dass über die gesamte Verweildauer des Werkstoffs in der Mundhöhle ein ausreichender Wirkstoffspiegel gewährleistet wird.

Als antibakteriell wirksame Substanzen werden neben den synthetisch hergestellten Antibiotika auch Stoffe verwendet, die sich aus Naturprodukten ableiten. Hierzu gehören u.a. Chitosan und dessen Derivate.

Die Schriften EP 0329098 B1, EP 0389629 B1, EP 1255576 B1 und EP 1237585 B1 offenbaren aushärtbare Pasten aus verschiedenen Oxiden bzw. Phosphaten und Chitosan als Bindemittel, wobei das Chitosan durch die basischen Eigenschaften der Oxide in seiner Löslichkeit verringert wird. Die beschriebene Anwendung im dentalen Bereich bezieht sich auf Wurzelfüllungsmaterialien bzw. aufgrund der fehlenden Stabilität gegen den pH-Wert im Mundbereich lediglich auf provisorische Füllungswerkstoffe.

Aus der japanische Schrift 02102165 A ist eine Chitosan und Hydroxylapatit enthaltende Masse bekannt, die jedoch als Keramik erst nach einer Sinterung zum Einsatz kommen kann.

Der Nachteil dieser Lösung ist, dass bei der Sinterung die als Binder wirkenden, organischen Bestandteile pyrolysiert werden.

Die Schriften EP 0287105 B1 und EP 1296726 B1 offenbaren ein knochenbildendes Implantatmaterial aus einem Glycosaminoglycan mit kationischen Polymeren als Matrixsubstanzen, in die Füllstoffpartikel knochenähnlicher Zusammensetzung eingelagert wird. Chitosan ist zwar ein Glycosaminoglycan, jedoch beschreiben die genannten Schrtiften ausdrücklich ein vom Körper resorbierbares Knochenersatzmaterial, das auch im Kieferbereich eingesetzt werden kann.

Die japanische Schrift 07157434 A beschreibt einen Proliferationsinhibitor für Bakterien der Mundhöhle, der durch Chitosan und dessen Derivate gebildet ist.

Aus der japanischen Schrift 10130427 A ist hierzu noch die Anlagerung von Metallionen an die Aminogruppen des Chitosans bzw. dessen Derivaten bekannt, wobei dieses System mit Hydroxylapatit eingesetzt wird.

Ein ähnliches Material aus Chitosanderivaten und Zinnfluorid wird in der japanischen Schrift 05000930 A offenbart.

Bis heute wird Chitosan nur in Verbindung mit bioresorbierbaren Füllstoffen, wie bspw. Calciumphosphat eingesetzt und dient als abbaubarer Knochenfüllstoff oder als provisorisches Zahnfüllmaterial. Chitosan wird dabei aufgrund seiner vom pH-Wert abhängigen Löslichkeit als Bindemittel verwendet.

Der Nachteil aller bekannten Werkstoffe ist, dass sie keine beständige antimikrobielle Wirkung für den langfristigen in-vivo Einsatz aufweisen.

Der Erfindung liegt daher die Aufgabe zugrunde, einen Werkstoff für den überwiegend medizinischen, langfristigen in-vivo Einsatz anzugeben, der die Nachteile des Standes der Technik vermeidet und ohne ein Wirkstoff-Releasing initiiert wird sowie nach dem Abtragen des Materials oder trotz Veränderung der Werkstoffform weiterhin bestehen bleibt. Darüber hinaus soll ein Verfahren zur Herstellung dieses Werkstoffs angegeben werden.

Gemäß der Erfindung wird diese Aufgabe durch die kennzeichnenden Merkmale des ersten Patentanspruchs gelöst und durch vorteilhafte Ausgestaltungen gemäß den Unteransprüchen ergänzt.

Das Wesen der Erfindung besteht darin, dass ein Werkstoff auf Polymerbasis bereitgestellt wird, wobei dieser bei einer medizinischen Anwendung in der Mundhöhle, bspw. als Füllung oder Zement, eine antimikrobielle / antibakterielle Wirkung während der gesamten Verweildauer entfaltet, ohne toxisch oder allergisch zu wirken. Dabei besteht diese Wirkung auch nach erfolgtem Materialabtrag oder nach erfolgter Beschädigung fort.

Vorteilhaft besteht der Werkstoff aus Füllkörpern in Form von Polymeren, Copolymeren, Compositen, Metallen, glasähnlichen Verbindungen, Keramik in Reinform oder als Mischungen dieser Materialien, die mit einer polymeren Sicht in Form von Polysacchariden oder deren Derivaten beschichtet ist, wobei diese polymeren Beschichtungen eine antimikrobielle Wirkung aufweisen und die beschichteten Füllköper von einer Matrix, bestehend aus einem weiteren Polymer, umgeben sind.

Besonders vorteilhaft ist dieses Polysaccarid Chitosan.

Gemäß der Erfindung wird das Polymer, bspw. in Form von Chitosan, durch eine Deacetylierung so modifiziert, dass das deacetylierte Polymer, bspw. das Chitosan, auf eine modifizierte Siliziumdioxid-Partikeloberfläche (endständige Aldehydgruppen auf der Partikeloberfläche) koppelbar ist und im Anschluss daran ein Ankopplung von 3-Vinylbenzaldehyd an die polymerbeschichteten Partikel erfolgen kann.

Diese antimikrobiell wirkende Beschichtung kann zusätzlich chemisch so modifiziert sein, dass Kohlenstoff-Kohlenstoff-(Doppel)-Bindungen eingeführt werden, die beim Aushärtungsprozess an der chemischen Reaktion (z.B. Polymerisation) mit teilnehmen.

Darüber hinaus kann die zusätzliche chemische Modifikation dazu dienen, das Dispersionsverhalten zu verändern, aktivierbare Startermoleküle (Initiatoren, die bspw. chemisch, thermisch oder unter UV-Licht aktivierbar sind) auf der Oberfläche zu immobilisieren bzw. für die chemische Reaktion (bspw. die. Polymerisation) notwendige oder zusätzliche Reaktionsbeschleuniger oder Regler zur Einstellung der Kettenlänge auf der Oberfläche zu immobilisieren.

Dieser so aktivierte Füllstoff wird in einer flüssigen Monomermischung, bspw. Bis-GMA, TEGDMA, UDMA, BPO, Campherchinone oder Ketonen, dispergiert, so dass der erfindungsgemäße Werkstoff erhalten wird.

Durch die erfindungsgemäßen Beschichtung der Polymerpartikel wird eine über längere Zeiträume anhaltende antibakterielle Wirkung erzeugt, wobei gleichzeitig die Verbindung zur Polymermatrix und die damit verbundene verbesserte Dispergierung des Partikel-Pulvers in der flüssigen Phase bewirkt wird

Bei dem Dispergieren reagiert die endständige Vinylgruppe der Partikel (aktivierten Füllstoffe) mit den Monomeren unter Aushärtung zu einer Polymermatrix. Der aktivierte Füllstoff ist somit in Folge der chemischen Bindung integrativer Bestandteil des erfindungsgemäßen Werkstoffs.

Die Erfindung wird nachstehend an Hand des Ausführungsbeispiels näher erläutert.

### 1. Deacetylierung des Chitosans:

Die Deacetylierung des Chitosans erfolgt gemäß dem bekannten Verfahren unter Rückfluss in Salzsäure. Das so deacetylierte Chitosan wird gemäß dem Stand der Technik durch ein Dialyseverfahren aufgereinigt und durch Gefriertrocknung in eine feste Form überführt.

### 2. Kopplung des deacetylierten Chitosans auf modifizierte Siliziumdioxid-Partikeloberflächen / Ankopplung von 3-Vinylbenzaldehyd:

Die Hydroxylgruppen von Siliziumdioxid-Partikeln werden mit 3-Aminopropyl-triethoxysilan in einer Mischung aus Ethanol/Wasser bei 45 °C umgesetzt.

Nach Aufreinigung der Partikel / der Füllkörper durch Spülen mit Ethanol werden die Aminogruppen mit Glutaraldehyd bei Raumtemperatur unter Bildung einer Schiffschen Base modifiziert und anschließend mit Wasser gespült. Dadurch erhält man eine endständige Aldehydgruppen auf den Siliziumdioxid-Partikeln / den Füllkörpern, die mit einer wässrigen Lösung aus deacetyliertem Chitosan bei Raumtemperatur umgesetzt wird.

Die mit Chitosan modifizierte Partikeloberfläche / Füllkörperoberfläche wird mit 3-Vinylbenzaldehyd umgesetzt. Dabei reagieren die überschüssigen Aminogruppen des Chitosans mit dem 3-Vinylbenzaldehyd unter Bildung einer Schiffschen Base. Die Partikel / Füllkörper werden vom nicht kovalent gebundenen 3-Vinylbenzaldehyd durch mehrfaches Spülen mit Wasser gereinigt und anschließend getrocknet.

Durch diesen Verfahrensablauf besitzt / besitzen das Pulver / die Füllkörper kovalent gebundenes Chitosan auf seiner / ihre Oberfläche, dessen Aminogruppen zum Teil durch die Reaktion mit 3-Vinylbenzaldehyd chemisch modifiziert sind.

Zur Herstellung des Werkstoffs wird/werden das modifizierte Pulver / die Füllkörper in die Monomermischung (z.B. Bis-GMA, TEGDMA, UDMA, BPO, Campherchinone oder Ketone) dispergiert. Die endständige Vinylgruppe der Partikel / Füllkörper reagiert mit den Monomeren bei der Reaktion (Aushärtung des Füllmaterials) zur Polymermatrix. Der aktivierte Füllstoff ist somit in dem Polymer chemisch eingebunden und bildet mit diesem den erfindungsgemäßen Werkstoff.

### 3. Der Nachweis für die antibakterielle Wirkung wurde über Bakterienanlagerungstests erbracht.

Zum Nachweis der chemischen Integration der Füllkörper an die Polymermatrix werden dynamisch-mechanische Tests (DMA) sowie Biegeuntersuchungen durchgeführt.

Dazu werden Probekörper (bspw. in Form von Plättchen) unter Verwendung des erfindungsgemäßen Werkstoffs hergestellt.

Als Referenz kann bspw. ein Werkstoff mit nicht-modifizierten Pulver /Füllkörpern gemäß dem Stand der Technik eingesetzt. Die Anteile des Pulvers / der Füllkörper im Füllmaterial liegen dabei bekanntermaßen bei 20-30 Vol%.

Die Probekörper werden einer Suspension von Bakterien ausgesetzt (bspw. *Streptococcus sanguis*)*.* Die Bakterien haben somit die Möglichkeit sich auf der Probenoberfläche anzulagern und zu vermehren.

Nach 36 Stunden werden die oberflächlich auftretenden Bakterienzahlen bei dem erfindungsgemäßen Werkstoff quantitativ vermittels Fluoreszenzverfahren und Rasterelektronenmikroskop erfasst und mit den Bakterienzahlen der Referenz verglichen.

Alle in der Beschreibung und den nachfolgenden Ansprüchen dargestellten Merkmale können sowohl einzeln als auch in beliebiger Kombination miteinander erfindungswesentlich sein.

## Patentansprüche

1. Werkstoff für den überwiegend medizinischen, langfristigen in-vivo Einsatz bestehend aus einer Polymermatrix und antimikrobiellen Füllkörpern, **dadurch gekennzeichnet, dass** die Füllkörper mit einem antimikrobiell wirksamen Polysaccharid beschichtete SiliziumdioxidPartikel sind, welche endständige Vinylgruppen tragen, durch die die Füllkörper in der Polymermatrix eingebunden sind.

2. Werkstoff gemäß Anspruch 1, **dadurch gekennzeichnet, dass** die Polymerbeschichtung der Partikel aus Chitosan besteht.

3. Werkstoff gemäß Anspruch 1, **dadurch gekennzeichnet, dass** die Polymermatrix aus Bis-GMA, TEGDMA, UDMA, BPO, Campherchinone oder Ketonen gebildet ist.

4. Verfahren zur Herstellung eines Werkstoffs gemäß Anspruch 2 oder 3 umfassend folgende Schritte:
• Reaktion von 3-Aminopropyl-triethoxysilan mit Hydroxylgruppen-tragenden Siliziumdioxid-Partikeln in einer Mischung aus Ethanol/Wasser zur Ausbildung von Aminogruppen,
• Reaktion von Glutaraldehyd unter Bildung einer Schiffschen Base zur Ausbildung von endständigen Aldehydgruppen auf den Siliziumdioxid-Partikeln,
• Beschichtung dieser Partikel mit deacetyliertem Chitosan,
• Reaktion der beschichteten Partikel mit 3-Vinylbenzaldehyd zur chemischen Modifikation,
• Dispersion der Chitosan-beschichteten, chemisch modifizierten Partikel in einer Monomermischung unter Ausbildung einer aushärtbaren Phase zur Generierung der Polymermatrix.

## Claims

1. Material consisting of a polymer matrix and antimicrobial filling agents for primarily medical, long-term in vivo purposes, wherein the filling agents are silicon dioxide particles coated by an antibiotic polysaccharide, and said particles carry terminal vinyl groups by means of which the filling agents are incorporated into the polymer matrix.

2. Material in accordance with claim 1, wherein the polymer coating of the particles comprises chitosan.

3. Material in accordance with claim 1, wherein the polymer matrix is formed monomers selected from bis-GMA, TEGDMA, UDMA, BPO, campherchinone or ketones.

4. Method for producing a material according claim 2 or 3, comprising the following steps:
• reacting 3-aminopropyl-triethoxysilan with hydroxyl group-carrying silicon dioxide particles in a mixture of ethanol/water thereby to form amino groups on the particles,
• reacting glutaraldehyde with the amino groups, forming a Schiff base, thereby to form terminal aldehyde groups on the silicon dioxide particles,
• coating the particles having terminal aldehyde groups with deacetylated chitosan,
• reacting the coated particles with 3-vinyl benzaldehyde thereby to chemically modify the coating; and
• dispersing the chitosan-coated, chemically modified particles in a monomer mixture and then polymerizing the monomer matrix thereby to form a polymer matrix.

## Revendications

1. Matériau prévu pour l'utilisation principalement médicale, in vivo à long terme, et procédé de réalisation, qui se compose d'une matrice en polymère et des corps de remplissage antimicrobiens, est **caractérisé en ce que** les corps de remplissage sont des particules de dioxyde de silicium revêtues avec un polysaccharide d'efficacité antimicrobienne, portant des groupes vinyliques terminaux, par lesquels les corps de remplissage sont intégrés dans la matrice en polymère.

2. Matériau suivant la revendication 1 est **caractérisé en ce que** le revêtement polymère des particules se compose de chitosan.

3. Matériau suivant la revendication 1 est **caractérisé en ce que** la matrice en polymère est formée de Bis GMA, TEGDMA, UDMA, BPO, quinones de camphre ou cétones.

4. Le procédé de fabrication d'un matériau suivant la revendication 2 ou 3 contenant les phases suivantes:
• Réaction de silane du triéthoxy 3-aminopropyl avec des particules de dioxyde de silicium portant des groupes hydroxyles dans un mélange eau-éthanol pour la formation des groupes amino,
• Réaction de glutaraldéhyde sous l'apparition d'une base de Schiff pour la formation des groupes aldéhydes terminaux sur les particules de dioxyde de silicium,
• Revêtement de ces particules avec chitosan désacétylé,
• Réaction des particules revêtues avec benzaldéhyde de 3-vinyle pour la modification chimique,
• Dispersion des particules revêtues de chitosan et chimiquement modifiées dans un mélange monomère sous formation d'une phase durcissable pour la génération de la matrice en polymère.
